# EUROPEAN PATENT SPECIFICATION

(11) **EP 2 997 823 B1**
(45) Date of publication and mention of the grant of the patent: **20.12.2017**
(21) Application number: 14380026.6
(22) Date of filing: 19.09.2014
(51) Int. Cl.: A01N 1/02, A61N 5/06

(54) **Procedure to improve sperm quality in mammals**
Verfahren Spermienqualität bei Säugetieren zu verbessern
Procédure pour améliorer la qualité du sperme chez les mammifères

(43) Date of publication of application: 23.03.2016
(73) Proprietor: Instruments Utils de Laboratori Geniul, SL, 08222 Terrassa, Barcelona (ES)
(72) Inventor: Codony Iglesias, Francesc, 08302 Mataró (Barcelona) (ES); Rodríguez Gil, Juan Enrique, 08271 Artés (Barcelona) (ES)
(74) Representative: Juncosa Miro, Jaime

(56) References cited:
- WO-A1-2011/051774
- HUSSEIN Z M ET AL: "The effect of diode laser and light emitting diode on the bacterial contamination of semen medium for artificial insemination", BIOLOGICALS, ACADEMIC PRESS LTD., LONDON, GB, vol. 36, no. 5, 1 September 2008 (2008-09-01), pages 303-307, XP024527515, ISSN: 1045-1056, DOI: 10.1016/J.BIOLOGICALS.2008.04.003 [retrieved on 2008-06-09]
- FIEDLER SVEN ET AL: "Incoherent broad-band cavity-enhanced absorption spectroscopy of liquids", REVIEW OF SCIENTIFIC INSTRUMENTS, AIP, MELVILLE, NY, US, vol. 76, no. 2, 10 January 2005 (2005-01-10), pages 23107-023107, XP012079157, ISSN: 0034-6748
- COHEN N ET AL: "LIGHT IRRADIATION OF MOUSE SPERMATOZOA: STIMULATION OF IN VITRO FERTILIZATION AND CALCIUM SIGNALS", PHOTOCHEMISTRY AND PHOTOBIOLOGY, WILEY-BLACKWELL PUBLISHING, INC, US, vol. 68, no. 3, 1 January 1998 (1998-01-01), pages 407-413, XP001050364, ISSN: 0031-8655, DOI: 10.1562/0031-8655(1998)068<0407:LIOMSS>2.3 .CO;2
- S. SHAHAR ET AL: "Light-mediated activation reveals a key role for protein kinase A and sarcoma protein kinase in the development of sperm hyper-activated motility", HUMAN REPRODUCTION, vol. 26, no. 9, 19 July 2011 (2011-07-19), pages 2274-2282, XP055164200, ISSN: 0268-1161, DOI: 10.1093/humrep/der232
- CORRAL-BAQUÉS M I ET AL: "Effect of 655-nm diode laser on dog sperm motility", LASERS IN MEDICAL SCIENCE, SPRINGER-VERLAG, LO, vol. 20, no. 1, 1 July 2005 (2005-07-01), pages 28-34, XP019361463, ISSN: 1435-604X
- J M Ocafia-Quero ET AL: "Biological effects of helium-neon (He-Ne) laser irradiation on acrosome reaction in bull sperm cells", ELSEVIER Journal of Photochemistry and Photobiology B: Biology, 1 January 1997 (1997-01-01), pages 294-298, XP055164620, Retrieved from the Internet: URL:http://ac.els-cdn.com/S101113449700072 9/1-s2.0-S1011134497000729-main.pdf?_tid=5 ea82320-a318-11e4-91e4-00000aacb362&acdnat =1422028759_f68b99f513289a0faef4c9e820aa90 eb [retrieved on 2015-01-23]
- CORRAL-BAQU PRG A(C)S M I ET AL: "The effect of low-level laser irradiation on dog spermatozoa motility is dependent on laser output power", LASERS IN MEDICAL SCIENCE, SPRINGER-VERLAG, LO, vol. 24, no. 5, 12 September 2008 (2008-09-12), pages 703-713, XP019725026, ISSN: 1435-604X
- J M Ocañ-Quero ET AL: "Helium-Neon (He-Ne) Laser Irradiation Increases the Incidence of Unreduced Bovine Oocytes During the First Meiotic Division In Vitro", Lasers Med Sci, 1 January 1998 (1998-01-01), pages 260-264, XP055164625, Retrieved from the Internet: URL:http://download.springer.com/static/pd f/463/art%3A10.1007%2Fs101030050005.pdf?au th66=1422029065_e91d36f837090ea85b7631ee41 117f05&ext=.pdf [retrieved on 2015-01-23]
- H. BREITBART ET AL: "Changes in calcium transport in mammalian sperm mitochondria and plasma membrane irradiated at 633 nm (HeNe laser)", JOURNAL OF PHOTOCHEMISTRY AND PHOTOBIOLOGY B: BIOLOGY, vol. 34, no. 2-3, 1 July 1996 (1996-07-01) , pages 117-121, XP055164626, ISSN: 1011-1344, DOI: 10.1016/1011-1344(95)07281-0
- N. IAFFALDANO ET AL: "The post-thaw irradiation of avian spermatozoa with He-Ne laser differently affects chicken, pheasant and turkey sperm quality", ANIMAL REPRODUCTION SCIENCE, vol. 142, no. 3-4, 1 November 2013 (2013-11-01), pages 168-172, XP055164627, ISSN: 0378-4320, DOI: 10.1016/j.anireprosci.2013.09.010
- IAFFALDANO N ET AL: "Improvement of stored turkey semen quality as a result of He-Ne laser irradiation", ANIMAL REPRODUCTION SCIENCE, ELSEVIER SCIENCE PUBLISHERS, AMSTERDAM, NL, vol. 85, no. 3-4, 1 February 2005 (2005-02-01), pages 317-325, XP027622652, ISSN: 0378-4320 [retrieved on 2005-02-01]
- H. BREITBART ET AL: "Changes in calcium transport in mammalian sperm mitochondria and plasma membrane irradiated at 633 nm (HeNe laser)", JOURNAL OF PHOTOCHEMISTRY AND PHOTOBIOLOGY B: BIOLOGY, vol. 34, no. 2-3, 1 July 1996 (1996-07-01) , pages 117-121, XP055164628, ISSN: 1011-1344, DOI: 10.1016/1011-1344(95)07281-0
- IAFFALDANO N ET AL: "The irradiation of rabbit sperm cells with HeNe laser prevents their in vitro liquid storage dependent damage", ANIMAL REPRODUCTION SCIENCE, ELSEVIER SCIENCE PUBLISHERS, AMSTERDAM, NL, vol. 119, no. 1-2, 1 May 2010 (2010-05-01) , pages 123-129, XP026912998, ISSN: 0378-4320, DOI: 10.1016/J.ANIREPROSCI.2009.10.005 [retrieved on 2009-10-24]

## Description

### Field of the Art

The present invention relates to a methodology for the treatment of mammalian seminal fluids which is not based on chemical reagents and has the objective of increasing fertility of the seminal fluid by means of, but not limited to, activating the motility of the spermatozoa contained in said fluid, their resistance to stress and generally improving spermatozoa viability.

This methodology is based on treatment by means of exposing a vessel containing the seminal fluid to an electromagnetic (visible light) radiation. The treatment conditions have been optimized to specific wavelength, time and dose values in an isothermal environment.

The present invention is encompassed in the field of assisted reproductive techniques in mammals.

This invention comprises a method in which all the variables of exposure have been verified and optimized, and equipment designed for carrying out this process on vessels, cannulas, syringes, test tubes or similar systems used in the steps prior to insemination.

### Prior State of the Art

Sperm viability, vigor and motility describe spermatozoon functionality to suitably move towards the ovule. These factors are directly related with sperm quality and much more decisive in achieving fertilization than the total sperm count. The spermatozoon having insufficient or unsuitable motility does not reach the ovule and this cannot be fertilized naturally.

Spermatozoon migration through the female reproductive tract (internal fertilization) or in synthetic fluids (external fertilization) to reach the ovule is key to successful fertilization. On the other hand, spermatozoon motility is also necessary to go through the extracellular matrices surrounding the ovule.

Spermatozoon motility is activated by specific physiological changes that can vary from one species to another. These changes include, for example, changes in pH, in calcium ion concentration and in specific ligands such as Camp.

Without technological intervention, spermatozoa with low vigor, non-motile or with abnormal motility will not fertilize the ovule; this is one of the main causes of subfertility or infertility.

In artificial insemination methods, semen suspension is introduced into the female reproductive tract. In the scope of infertility treatment in humans, there is a prior capacitation and selection of those spermatozoa with motility which are then introduced directly into the uterus.

Regardless of the type of mammalian spermatozoon to be treated, the object of this patent is to increase spermatozoa motility using non-chemical treatments based on exposure to specific conditions of visible light.

Today, there are different laboratory protocols used for making semen suspension, performing sperm capacitation, and if applicable, selecting cells having suitable motility. Generally, these suspensions are prepared at specific ionic and nutrient concentrations and at a specific pH.

Patent US 5,453,354 discloses a macromolecule of proteinaceous nature which activates sperm motility and the corresponding process for the preparation thereof by means of purifying the macromolecule from extracellular fluids.

Patent US 7,780,230 provides a composition comprising a cytoplasmic extract purified from eggs of *Xenopus laevis* which is capable of supporting activation of human sperm and complete replication of a human sperm genome.

Patent US 6,309,815 provides a method and a composition for extending the viability of immotile spermatozoa. The method comprises collecting the sperm and treating it with a storage buffer solution to substantially inhibit spermatozoa motility, then storing the semen for a period of time and at a specific temperature and finally reactivating the sperm to normal motility by means of mixing the inhibited spermatozoa with an activation buffer.

On the other hand, the response of ram and fish (tilapia) sperm to different types of light was published in 2005 [1], different types of response being detected depending on the type of sperm and of the type of light. The conclusions made were that the *in vitro* treatments of mammalian (ram) semen must be performed in the absence of light or under red light, in which a slight increase in motility was detected.

Studies analyzing the response of spermatozoon to visible light in general (the entire spectrum) and the possible cellular mechanisms involved have been published recently [2]. This paper concluded that light radiation for short periods of time caused significant increases in motility, without specifying a specific range of the visible spectrum (it refers to the entire spectrum) or the specific amounts of energy.

The first approach to the activation of mammalian spermatozoa motility with specific wavelengths was performed with dog semen [3, 4] and buffalo semen [5] at 655 nm and 532 nm, respectively. In the first case, a proportional relationship is reported between the type of light and the dose with the motility and in the second case, clear conditions of exposure by means of laser light systems are validated. This last paper suggests the possibility of using laser light as a technique for the *in situ* improvement of semen quality to optimize the artificial insemination conditions.

Although there is evidence suggesting that phototherapy may be applicable in artificial insemination therapies in mammals, most studies are not more than actions on semen suspensions and only provide confirmation of certain limited increase in motility, but without assessing if other important parameters for determining semen quality are affected and if that actually translates into an increase in the probability of pregnancy in field studies or the way it should be applied at the equipment level in real-life conditions.

Patent US 6 379 939 describes a method for increasing the fertilizing capacity of mammalian, particularly human, sperm cells and it proposes irradiating said sperm cells with light in the extended visible range (300 to 1000 nm) with an intensity of 1 to 1000 mW/cm² wherein the light is not produced by laser and for a period of time between 0.5 and 10 minutes.

Scientific document Hussein Z M et. al. "The effect of diode laser and light emitting diode on the bacterial contamination of semen medium for artificial insemination" describes an optical method developed for bacterial growth inhibition in semen containing extender medium using diode laser (DL) and commercial cheap light emitting diode (LED). Certain wavelengths and exposure times suitable for the process of artificial insemination are found to be optimum at reducing bacterial growth with a minimum significant effect on the semen motility and viability.

It is necessary to provide an alternative to the state of the art which covers the lack of detailed information while at the same time allows working with optical systems that are not based on laser, having clear operative drawbacks: price and possibility of implementation in *in situ* work equipment.

### Brief Description of the Invention

In view of the prior art and prior results, the present inventors have focused on the study of phototherapy as a way for improving semen quality, but establishing *in situ* protocols in human reproduction centers or animal farms. Prior laboratory results have been confirmed for such purpose and in real-life conditions, verifying: light cycles, light dose, time of exposure and devices to perform this treatment on the conventional containers for containing such fluids and under cooling or constant temperature conditions.

This invention describes the conditions for the treatment of mammalian sperm solutions with optical systems that are not based on laser. In this case, a specific range of wavelengths, times of exposure and time of use of the spermatozoa solution in which the motility is maximum are defined, optimum insemination capacity of the spermatozoa being thus assured.

For this purpose, the invention provides a method for increasing the fertilizing capacity of, for example mammalian, sperm cells which, like the known techniques, comprises irradiating said sperm cells with non-coherent red light.

However, the method of the invention is characterized in that the irradiation with non-coherent red light is performed in a discontinuous manner according to a pattern including at least one sequence of two irradiation periods of specific durations which are separated by an intermediate period of darkness of a specific duration, each of said specific periods of irradiation and darkness having a duration between 8 and 15 minutes, which produce very beneficial results for the desired purpose as confirmed by the inventors.

Said duration is preferably 10 minutes. Furthermore, the mentioned periods of exposure and darkness can have the same or different duration.

On the other hand, the red light can have a wavelength between 620 and 630 nm.

According to one embodiment, at least one semen sample including said sperm cells is diluted in a diluent, and the mentioned irradiation is applied on at least one portion of the diluted sample. Likewise, the at least one portion of the diluted sample or a non-diluted portion of said semen sample can also be introduced in a container having walls transparent to the wavelength of said irradiated red light and the irradiation can be applied on said container. The semen is kept in darkness as a result of the casing of the container.

According to one embodiment, the container is a test tube or microcentrifuge tube and will have a size suitable for the insemination treatment.

The diluted or non-diluted portion of semen sample is preferably kept refrigerated at least until the time immediately before the application of the red light irradiation thereon and/or during said application.

Likewise, the portion or portions of semen sample can be incubated in an isothermal medium at substantially 16-21°C after being subjected to the mentioned pattern of red light irradiations. Generally, the incubation is performed for different periods, after each of which the method comprises performing analytical controls on the portion or portions of semen sample for determining at least the state of functional viability and motility of the spermatozoa.

Once the treatment of the sperm cells has been performed according to the principles of this invention, it has been verified that the percentage of motile spermatozoa and their level of motility improved with respect to their prior baseline level, as has their stress resistance capacity.

Since all the factors which improve the viability and motility of motile spermatozoa are a key factor in a successful ovule fertilization process, this enhancement of cell viability and vigor will increase fertilization probabilities.

### Brief Description of the Drawings

The foregoing and other advantages and features will be better understood based on the following detailed description of several embodiments in reference to the attached drawings which must be interpreted in an illustrative and non-limiting manner, in which:
Figure 1 is an example of small samples in 2 ml tubes;
Figure 2 is an example of diluted samples in 50 ml containers.

### Detailed Description of several Embodiments

To confirm the prior evidence and the actual magnitude of phototherapy impact for improving the different descriptive parameters of sperm cell motility-viability and sperm cell resistance to environmental stress (this being considered as 37°C in times of more than 1 hour), different laboratory studies were conducted using porcine semen as a mammalian model.

Work was performed with porcine spermatozoa because they are a material that can be easily obtained and because they are a good model for extrapolating results to other mammals, including humans.

Work was performed with samples from 15 healthy adult males aged 2-3 and from 5 different breeds (Pietrain, Duroc, Large White, Belgian Landrace and British Landrace). These samples were obtained by means of manual stimulation, immediately diluted in a commercial diluent to a final sperm concentration of 3x10⁷ spermatozoa/ml in a final volume of 90 mL and kept at 16-17°C.

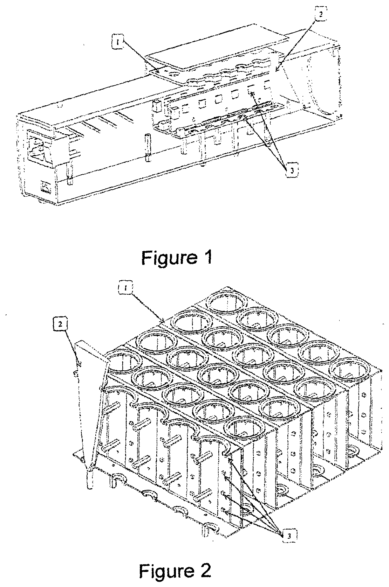

Two aliquots of 1.5 mL were deposited in microcentrifuge tubes, one aliquot was kept in incubation at 16-17°C as control and the other aliquot was exposed to different photostimulation programs by means of the device described in Figure 1.

The photostimulation programs implemented are detailed below:
(A) 10 minutes of exposure to red light, 10 minutes of rest and 10 minutes of exposure to red light.
(B) 15 minutes of exposure to red light, 10 minutes of rest and 15 minutes of exposure to red light.
(C) 20 minutes of exposure to red light, 10 minutes of rest and 20 minutes of exposure to red light.

Once the treatment has been performed, both the different control aliquots and the treated aliquots were introduced in a thermostatic bath at 37°C, being incubated for different times (15, 30, 60 and 90 minutes) after which analytical controls were performed for determining the state of functional viability and motility.

The analytical controls were:
1) the estimation of the percentages of viability and of structurally intact acrosomes by means of double staining with Trypan blue/Giemsa [6]
2) Sperm motility analysis by means of computer-aided motility analysis (CASA) measuring the following parameters: curvilinear velocity (VCL), straight-line velocity (VSL), average path velocity (VAP), linearity index (LIN), straightness index (STR), oscillation index (WOB), mean amplitude of lateral head displacement (ALH), mean best cross frequency (BCF).

The results of 5 independent tests were statistically evaluated by means of the log transformation of the data that did not follow normal distribution. Significant differences (p<0.05) were determined by means of the LSMEANS method of the SAS statistics package.

**Table 1. Experiment 1. Significant differences (*) at 90 minutes between controls and treatments A, B and C**

| Test | Treatment | | |
|---|---|---|---|
| | A | B | C |
| % Viability | * | * | |
| % Altered acrosomes | # | # | |
| % Total motility | * | | * |
| VCL | * | * | * |
| VSL | * | * | |
| VAP | * | * | * |
| LIN | * | * | |
| STR | * | * | * |
| WOB | | * | |
| ALH | | * | * |
| BCF | * | | |
| * greater than control # less than control | | | |

These works determined that a dose (time of exposure to the irradiation of non-coherent red light) of excessive duration did not have any effect or the result thereof was not optimum, and that the positive response, in terms of improvement in motility-viability, was strongly related with the combination of at least 2 exposures of non-coherent red light with an intermediate period of darkness of well determined durations in both cases. Generally, treatments A and B showed a clear protective effect, substantially improving sperm survival, the values obtained in treatment A being of a higher level than in treatment B. So treatment A was considered the best from all the treatments evaluated until then and was considered the starting point for application in real-life cases of assisted reproduction in mammals.

For the purpose of verifying if the discontinuous treatment (with an intermediate rest period) had a clear improvement over a treatment that is equivalent but with light dosed in a continuous manner, the following treatment protocols were assessed with the same materials and methods described above.

The photostimulation programs were:
(treatment A again and an unexposed control)
(D) 10 minutes of exposure to red light,
(E) 20 minutes of exposure to red light
(F) 30 minutes of exposure to red light
(G) 45 minutes of exposure to red light

**Table 2. Experiment 2. Significant differences (*) at 90 minutes between controls and treatments A, D, E, F and G**

| Test | Treatment | | | | |
|---|---|---|---|---|---|
| | A | D | E | F | G |
| % Viability | * | | | | |
| % Altered acrosomes | # | | | | |
| % Total motility | * | | # | # | # |
| VCL | * | * | | | |
| VSL | * | | # | # | # |
| VAP | * | | | | # |
| LIN | * | | # | # | |
| STR | * | | | | |
| WOB | | | | | |
| ALH | | | | | # |
| BCF | * | * | * | * | * |

| | | | | | |
|---|---|---|---|---|---|
| * greater than control # less than control | | | | | |

Based on the conclusions of the results shown in Tables I and 2, it is deduced that the discontinuous treatments have a substantial improvement over the properties of the mammalian semen (using the semen from different species of pig as a model) and in contrast, a continuous treatment worsens the properties of the semen, these properties being key to fertilization.

It is inferred from the foregoing that controlled photostimulation with non-coherent red LED light, for example at a wavelength of 620-630 nm, in a 10-10-10 (10 minutes of irradiation, followed by 10 minutes of rest and 10 more final minutes of irradiation) pattern of stimulation significantly improve the thermal stress resistance of porcine spermatozoa previously diluted in a commercial diluent cooled at 16-17°C routinely used in artificial insemination in farms.

This pattern of photostimulation also leads to an improvement in the capacity of these spermatozoa to achieve progesterone-induced *in vitro* acrosome reaction. These *in vitro* results suggested that photostimulation has a significant effect on the spermatozoa, improving not only the capacity to resist environmental stress, but also the capacity to penetrate the ovule. Both effects may have, as a whole and as an end result, an improvement in the fertilizing capacity of the photostimulated spermatozoa, with subsequent improvement in the results of *in vivo* fertility and prolificacy, particularly under conditions in which the environmental stress may reduce said fertility and prolificacy.

An example of application would be in a pig farm where ambient temperature is acting to the detriment of the reproductive capacity.

A second example of application would be in the scope of human reproduction where the lack of motility due to environmental and/or organic causes is related with male infertility.

Since pigs are animals that are metabolically similar to humans and are accepted as models in different fields of medicine, and since the practical demonstration of the second example is ethically unviable without at least having been demonstrated beforehand with an animal model, the validity of this approach in mammals has been verified using pigs as models.

In a study conducted in a breeding farm following the photostimulation method described above with respect to commercial semen doses and using the equipment described above (Figure 2), 7 different batches of females in heat, in total n=195, were treated by post-cervical insemination. In a manner parallel to each batch of females treated with photostimulated semen suspension (n=78), a second batch was inseminated with untreated suspensions (n=117). The results can be seen in the following table:

**Table 3. Percentages of fertility for the control group and the group inseminated with photostimulated commercial doses.**

| Batch | Control | | | Photostimulated semen doses |
|---|---|---|---|---|
| | n | % fertility | n | % fertility |
| 1 | 15 | 93.3 | 13 | 100.0 |
| 2 | 25 | 96.00 | 10 | 100.0 |
| 2 | 18 | 100.00 | 13 | 100.0 |
| 4 | 14 | 92.85 | 13 | 92.3 |
| 4 | 15 | 100.00 | 12 | 100.0 |
| 6 | 16 | 93.33 | 12 | 100.0 |
| 7 | 16 | 93.75 | 11 | 90.91 |
| 8 | 37 | 91.91 | 19 | 94.74 |
| 9 | 19 | 84.23 | 14 | 100 |
| Total | 175 | 93.93 | 117 | 97.55 |

| | | | | |
|---|---|---|---|---|
| *n, number of pregnant females* *%, percentage of fertility with respect to the total of the group* | | | | |

The fertility results with a 95% confidence interval are (90.85-97.01)% for the controls and (95,07-100)% for the treated females. A statistical mean comparison test (a one-tailed paired t-test) gives p=0.0448 demonstrating that there are significant differences between the 2 groups despite the higher percentages of fertility in the control group denoting that the study has been conducted in very efficient breeding farms with a high percentage of fertility .

It must be highlighted that once pregnancy ends, a difference of 2 females with positive fertilization in a batch of 100 females is equivalent (with an average of 8 offsprings per individual) to a difference of at least 16 offsprings per batch which has a clear multiplicative effect on the livestock yield.

Data of the number of live births by gestated female with a 95% CI were 13.73 (12.82-14.45) for the control group and 14.31 (13.29-15.33) for those treated with photostimulated semen. These results demonstrate that once pregnant, the use of photostimulated semen does not reduce the prolificacy thereof.

The scope of protection of the invention is defined by the following claims.

### References

[1] T. Zan-Bar, B. Bartoov, R. Segal, R. Yehuda, R. Lavi, Dr. R. Lubart, and R.R. Avtalion. Photomedicine and Laser Surgery. 2005, 23(6): 549-555. doi:10.1089/pho.2005.23.549.
[2] Shahar S, Wiser A, Ickowicz D, Lubart R, Shulman A, Breitbart H. Hum. Reprod. (2011) 26 (9): 2274-2282. doi: 10.1093/humrep/der232
[3] Corral-Baqués MI, Rivera MM, Rigau T, Rodríguez-Gil JE, Rigau J.Lasers Med Sci. 2009 Sep;24(5):703-13.
[4] Corral-Baqués MI, Rigau T, Rivera M, Rodríguez JE, Rigau J.Lasers Med Sci. 2005;20(1):28-34
[5] Abdel-Salam Z, Dessouki SH, Abdel-Salam SA, Ibrahim MA, Harith MA. Theriogenology. 2011 Apr 1; 75(6):988-94.
[6] Rodríguez-Gil, J.E. and Rigau, T 1995 An. Rep. Sci., 39: 141-146.

## Claims

1. A method for increasing the fertilizing capacity of sperm cells, which comprises irradiating said sperm cells with non-coherent red light, **characterized in that** said irradiation is performed in a discontinuous manner with non-coherent red light according to a pattern including at least one sequence of two periods of sperm cell irradiation of a specific duration which are separated by an intermediate period of darkness of a specific duration, where each of said specific periods of irradiation and darkness has a duration between 8 and 15 minutes.

2. The method according to claim 1, wherein the duration of said two periods of irradiation and of the intermediate period of darkness is the same.

3. The method according to claim 1, where each of said specific periods of irradiation and darkness has a duration of 10 minutes, and the duration of said two periods of irradiation and of the intermediate period of darkness is the same.

4. The method according to the claim 1, wherein said periods of irradiation and darkness are of different duration.

5. The method according to any one of the preceding claims, where said red light has a wavelength between 620 and 630 nm.

6. The method according to any one of the preceding claims, which comprises diluting in a diluent at least one semen sample including said sperm cells, and applying said irradiation on at least one portion of said diluted sample.

7. The method according to claim 6, which comprises introducing said at least one portion or a non-diluted portion of said semen sample in a container having walls transparent to the wavelength of said irradiated red light and applying said irradiation on said container.

8. The method according to claim 7, where said container is a test tube or microcentrifuge tube having a suitable size for human or animal reproduction.

9. The method according to claims 6 to 8, which comprises keeping said at least one diluted or non-diluted portion of semen sample refrigerated at least until the time immediately before the application of the red light irradiation thereon and/or during said application.

10. The method according to any one of claims 6 to 9, which comprises incubating in an isothermal medium at substantially 16-21°C the portion or portions of semen sample after being subjected to said pattern of red light irradiations.

11. The method according to claim 10, **characterized in that** it comprises performing said incubation for different periods, after each of which the method comprises performing analytical controls on the portion or portions of semen sample for determining at least the state of functional viability and motility of the spermatozoa.

12. The method according to any one of the preceding claims, where said sperm cells are mammalian sperm cells.

## Patentansprüche

1. Verfahren zur Verbesserung der Befruchtungsfähigkeit von Samenzellen, welches das Bestrahlen der genannten Samenzellen mit nicht-kohärentem rotem Licht umfasst, **dadurch gekennzeichnet, dass** die genannte Bestrahlung diskontinuierlich mit nicht-kohärentem rotem Licht gemäß einem Muster beinhaltend mindestens eine Sequenz von zwei Zeiträumen von Bestrahlung der Samenzellen mit einer spezifischen Dauer, welche von einem Zwischenzeitraum von Dunkelheit mit einer spezifischen Dauer getrennt sind, durchgeführt wird, wobei jeder der spezifischen Zeiträume von Bestrahlung und Dunkelheit eine Dauer zwischen 8 und 15 Minuten hat.

2. Verfahren nach Anspruch 1, wobei die Dauer der genannten zwei Zeiträume von Bestrahlung und des Zwischenzeitraums von Dunkelheit gleich ist.

3. Verfahren nach Anspruch 1, wobei jeder der genannten spezifischen Zeiträume von Bestrahlung und Dunkelheit eine Dauer von im Wesentlichen 10 Minuten hat, und die Dauer der genannten zwei Zeiträume von Bestrahlung und des Zwischenzeitraums von Dunkelheit gleich ist.

4. Verfahren nach Anspruch 1, wobei die genannten Zeiträume von Bestrahlung und Dunkelheit eine unterschiedliche Dauer haben.

5. Verfahren nach einem der vorhergehenden Ansprüche, wobei das genannte rote Licht eine Wellenlänge zwischen 620 und 630 nm hat.

6. Verfahren nach einem der vorhergehenden Ansprüche, welches das Verdünnen in einem Verdünnungsmittel mindestens einer Samenprobe beinhaltend die genannten Samenzellen, und das Anwenden der genannten Bestrahlung auf mindestens einem Teil der genannten verdünnten Probe umfasst.

7. Verfahren nach Anspruch 6, welches das Einführen des genannten mindestens einen Teils oder eines nicht-verdünnten Teils der genannten Samenprobe in einen Behälter, welcher Wände aufweist, die gegenüber der Wellenlänge des genannten bestrahlten roten Lichtes transparent sind, und das Anwenden der genannten Bestrahlung auf dem genannten Behälter umfasst.

8. Verfahren nach Anspruch 7, wobei der genannte Behälter ein Reagenzglas oder ein Mikrozentrifugenröhrchen ist, welches eine geeignete Größe für die menschliche oder tierische Fortpflanzung hat.

9. Verfahren nach den Ansprüchen 6 bis 8, welches das Kühlhalten des genannten mindestens einen verdünnten oder nicht-verdünnten Teils der Samenprobe mindestens bis zum Zeitpunkt unmittelbar vor der Anwendung der Bestrahlung mit rotem Licht darauf und/oder während der genannten Anwendung umfasst.

10. Verfahren nach einem der Ansprüche 6 bis 9, welches das Inkubieren in einem isothermischen Mittel bei im Wesentlichen 16-21ºC des Teils oder der Teile der Samenprobe umfasst, nach dem Aussetzen derselben dem genannten Muster von Bestrahlungen mit rotem Licht.

11. Verfahren nach Anspruch 10, **dadurch gekennzeichnet, dass** es das Durchführen der genannten Inkubation während unterschiedlicher Zeiträume umfasst, wobei nach jedem derselben das Verfahren das Durchführen von analytischem Kontrollen auf dem Teil oder den Teilen der Samenprobe umfasst, zum Bestimmen mindestens des Zustands der funktionellen Lebensfähigkeit und Beweglichkeit der Spermatozoen.

12. Verfahren nach einem der vorhergehenden Ansprüche, wobei die genannten Samenzellen Samenzellen eines Säugetiers sind.

## Revendications

1. Une méthode pour accroître la capacité de fertilité des spermatozoïdes, qui comporte l'irradiation de ces spermatozoïdes avec de la lumière rouge non cohérente, **caractérisée en ce que** cette irradiation est effectuée de façon discontinue avec de la lumière rouge non cohérente conformément à un patron comportant au moins une séquence de deux périodes d'irradiation des spermatozoïdes d'une durée spécifique qui sont séparées par une période intermédiaire d'obscurité d'une durée spécifique, dans laquelle chacune de ces périodes spécifiques d'irradiation et d'obscurité a une durée de 8 à 15 minutes.

2. La méthode conformément à la revendication 1, dans laquelle la durée de ces deux périodes d'irradiation et de la période intermédiaire d'obscurité est la même.

3. La méthode conformément à la revendication 1, dans laquelle chacune des périodes spécifiques d'irradiation et d'obscurité a une durée de 10 minutes et la durée de ces deux périodes d'irradiation et de la période intermédiaire d'obscurité est la même.

4. La méthode conformément à la revendication 1, dans laquelle ces périodes d'irradiation et d'obscurité ont des durées différentes.

5. La méthode conformément à une quelconque des revendications précédentes, dans laquelle cette lumière rouge a une longueur d'onde de 620 à 630 nm.

6. La méthode conformément à une quelconque des revendications précédentes qui comporte diluer dans un diluent au moins un échantillon de sperme comprenant ces spermatozoïdes et en appliquant cette irradiation sur au moins une portion de cet échantillon dilué.

7. La méthode conformément à la revendication 6, qui comporte l'introduction de cette au moins une portion ou d'une portion non diluée de cet échantillon de sperme dans un récipient ayant des parois transparentes à la longueur d'ondes de cette lumière rouge irradiée et l'application de cette irradiation sur ce récipient.

8. La méthode conformément à la revendication 7, dans laquelle ce récipient est une éprouvette ou un tube de microcentrifugeuse ayant une taille appropriée pour la reproduction humaine ou animale.

9. La méthode conformément aux revendications 6 à 8, qui comporte conserver cette au moins une portion diluée ou non diluée d'échantillon de sperme réfrigérée au moins jusqu'au moment immédiatement avant l'application de l'irradiation de lumière rouge sur elle et/ou durant cette application.

10. La méthode conformément à une quelconque des revendications 6 à 9, qui comporte l'incubation dans un milieu isotherme à sensiblement 16-21ºC la portion ou les portions d'échantillon de sperme après qu'elle ait suivi ce schéma d'irradiations de lumière rouge.

11. La méthode conformément à la revendication 10, **caractérisée en ce qu'**elle comprend effectuer cette incubation pendant des périodes différentes, après chacune desquelles la méthode comporte effectuer des contrôles analytiques sur la portion ou les portions d'échantillon de sperme pour déterminer au moins l'état de viabilité fonctionnelle et la mobilité des spermatozoïdes.

12. La méthode conformément à une quelconque des revendications précédentes, dans laquelle les spermatozoïdes sont des spermatozoïdes de mammifères.
